# EUROPEAN PATENT APPLICATION

(11) **EP 4 442 803 A1**
(43) Date of publication of application: **09.10.2024**
(21) Application number: 22901748.8
(22) Date of filing: 30.11.2022
(51) Int. Cl.: C12M 1/34, C12M 1/00, B01L 3/02

(54) **ENDOSCOPIC PIPETTE AND OBSERVATION SYSTEM**

(30) Priority: 30.11.2021 KR 20210168532
(71) Applicant: Industry-Academic Cooperation Foundation, Yonsei University, Seoul 03722 (KR)
(72) Inventor: KIM, Baek Gil, Seoul 04305 (KR); CHO, Nam Hoon, Seoul 06098 (KR); JANG, Yeonsue, Seoul 04305 (KR); KANG, Suki, Paju-si Gyeonggi-do 10916 (KR)
(74) Representative: Schmidt, Christian
(86) International application number: PCT/KR2022/019161
(87) International publication number: WO 2023/101390

(57) **Abstract**

The present invention provides an endoscopic pipette and an observation system, the endoscopic pipette is configured to remove a culture medium accommodated in a culture vessel, in which spheroids are cultured, and observe the spheroids in the culture vessel, and the endoscopic pipette includes a tip unit configured to be inserted into the culture vessel to extract the culture medium, an accommodation unit configured to accommodate the culture medium extracted by the tip unit, a camera unit provided between the tip unit and the accommodation unit, disposed adjacent to the tip unit, and configured to capture an image of an interior of the culture vessel, and a power unit provided in a partial space of the accommodation unit and configured to provide power to the camera unit.

## Description

### [Technical Field]

The present invention relates to an endoscopic pipette and an observation system, and more particularly, to an endoscopic pipette and an observation system, which are capable of identifying states of spheroids in a culture vessel, which is difficult to visibly identify, and detecting motions of the spheroids during a process of extracting a culture medium, thereby preventing the spheroids from being lost.

### [Background Art]

Spheroids, which are produced by culturing cells, are too small to visibly observe sizes, shapes, and changes thereof. Therefore, microscopes may be used to observe the sizes, shapes, and changes of the spheroids.

However, a culture medium in a culture vessel in which spheroids are cultured, needs to be periodically replaced. A waste culture medium in the culture vessel, in which the spheroids are cultured, may be extracted, and a new culture medium may be supplied, such that the spheroids may be consistently grown to a desired degree.

However, when the spheroids are moved into a tip by a suction force applied to the tip to extract the culture medium during the process of extracting the waste culture medium, the culture medium may be lost, and the spheroids may be damaged while moving into the tip, which may result in wasted culture time.

Therefore, a method has been proposed to consistently and smoothly identify cultured states of spheroids and minimize a loss of the spheroids during the process of extracting the culture medium, and a means for implementing the method is required.

### [Disclosure]

### [Technical Problem]

The present invention has been made in an effort to solve the above-mentioned problem in the related art, and an object of the present invention is to identify states of spheroids in a culture vessel, which is difficult to visibly identify, and detect motions of the spheroids during a process of extracting a culture medium, thereby preventing the spheroids from being lost.

Technical problems of the present invention are not limited to the aforementioned technical problems, and other technical problems, which are not mentioned above, may be clearly understood by those skilled in the art from the following descriptions.

### [Technical Solution]

In order to achieve the above-mentioned object, the present invention provides an endoscopic pipette and an observation system, the endoscopic pipette is configured to remove a culture medium accommodated in a culture vessel, in which spheroids are cultured, and observe the spheroids in the culture vessel, and the endoscopic pipette includes: a tip unit configured to be inserted into the culture vessel to extract the culture medium; an accommodation unit configured to accommodate the culture medium extracted by the tip unit; a camera unit provided between the tip unit and the accommodation unit, disposed adjacent to the tip unit, and configured to capture an image of an interior of the culture vessel; and a power unit provided in a partial space of the accommodation unit and configured to provide power to the camera unit.

In this case, the accommodation unit may be divided into a first space in which the culture medium introduced through the tip unit is accommodated, a second space in which the power unit is positioned, and a third space in which the camera unit is positioned.

In this case, the second space may be disposed adjacent to the first space in which the culture medium is accommodated, and the second space may be elongated in a longitudinal direction of the accommodation unit.

Further, the power unit may include: a battery part configured to store electric power and provide the electric power to the camera unit; a switch part configured to operate the battery part; and a transmission part provided in the second space, disposed adjacent to the camera unit, and configured to transmit the image captured by the camera unit to the outside.

In addition, the second space may be divided into three or more spaces so that the battery part, the switch part, and the transmission part are positioned in the separated spaces.

Further, the defined space of the second space, in which the battery part is positioned, may be formed to be openable and closable so that the battery part is replaced depending on the amount of electric power remaining in the battery part.

Meanwhile, the third space may be disposed below the first space, and the tip unit may be coupled to penetrate the third space toward the first space.

In this case, the tip unit may include: a through portion configured to penetrate the third space and having a constant diameter; and an extraction portion extending to the outside of the third space so that a diameter thereof decreases from the through portion toward the culture vessel, the extraction portion being inserted into the culture vessel to extract the culture medium.

Meanwhile, the camera unit may include: an image capturing part configured to capture an image of the interior of the culture vessel; and an adjustment part in which the image capturing part is positioned, the adjustment part being configured to move the image capturing part in a rotation direction.

In this case, the adjustment part may include: a rotary member formed to surround a periphery of the image capturing part and partially exposed to the outside of the third space, the rotary member configured to move the image capturing part while rotating; a casing member having one end at which the rotary member is positioned, the casing member being configured to rotate in a rotation direction of the rotary member and configured such that a part of the image capturing part is inserted into the casing member; and a spring member having one end and the other end respectively coupled to the image capturing part and the casing member, the spring member being configured to move the image capturing part toward the culture vessel in accordance with the rotation of the rotary member.

In addition, the camera unit may be provided as a pair of camera units positioned with the tip unit interposed therebetween.

Further, the camera unit may include an angle adj ustment part positioned between the pair of adjustment parts and configured to change an angle of the image capturing part while changing a distance between the other ends of the adjustment parts.

In addition, the angle adjustment part may include: a serrated member having one end positioned outside the third space, and the other end positioned in the third space, the serrated member having a plurality of protrusions formed along a periphery of the other end; and a pair of distance members provided with the serrated member interposed therebetween, the pair of distance members having grooves corresponding to the protrusions of the serrated member and configured to be moved by a rotation of the serrated member.

Meanwhile, the present invention provides an observation system, which is configured to observe spheroids accommodated in a culture vessel, the observation system including: the endoscopic pipette; and a display configured to receive an image captured by the endoscopic pipette and display the image.

In this case, the endoscopic pipette may transmit the image, which is captured by the image capturing part, to the display through the transmission part.

In addition, the transmission part may be connected to the display through Bluetooth.

Further, the observation system may include a correction unit configured to correct a pair of images captured by a pair of image capturing parts so that the pair of images is visibly observed through binocular disparity.

### [Advantageous Effects]

The endoscopic pipette and the observation system of the present invention provided to achieve the above-mentioned object may identify the states of the spheroids in the culture vessel, which is difficult to visibly identify, and detect the motions of the spheroids during the process of extracting the culture medium, thereby preventing the spheroids from being lost.

The effects of the present invention are not limited to the aforementioned effects, and other effects, which are not mentioned above, will be clearly understood by those skilled in the art from the claims.

### [Description of Drawings]

A detailed description of the exemplary embodiments of the present application to be described below as well as the summary explained above will be understood well when reading the detailed description and the summary with reference to the accompanying drawings.

The exemplary embodiments are illustrated in the drawings for the purpose of exemplifying the present invention.

However, it should be understood that the present application is not limited to the illustrated exact arrangement and means.
FIG. 1 is a view for explaining an overall configuration of an endoscopic pipette and an observation device of an observation system according to an embodiment of the present invention.
FIG. 2 is a view for explaining a tip unit of the endoscopic pipette and the observation system according to the embodiment of the present invention.
FIG. 3 is a view for explaining an accommodation unit of the endoscopic pipette and the observation system according to the embodiment of the present invention.
FIG. 4 is a view for explaining a camera unit of the endoscopic pipette and the observation system according to the embodiment of the present invention.
FIG. 5 is a view for explaining an angle of a camera unit of the endoscopic pipette and the observation system according to the embodiment of the present invention.
FIG. 6 is a view for explaining a pair of camera units of the endoscopic pipette and the observation system according to the embodiment of the present invention.
FIG. 7 is a view for explaining an angle adjustment part of the endoscopic pipette and the observation system according to the embodiment of the present invention.
FIG. 8 is a view for explaining a serrated member and a distance member of the endoscopic pipette and the observation system according to the embodiment of the present invention.
FIG. 9 is a view for explaining a power unit of the endoscopic pipette and the observation system according to the embodiment of the present invention.
FIG. 10 is a view for explaining the endoscopic pipette and the observation system according to the embodiment of the present invention.

### <Explanation of Reference Numerals and Symbols>

- C:: Spheroid
- W:: Culture vessel
- 10:: Pipette
- 12:: Transmission part
- 20:: Display
- 100:: Tip unit
- 120:: Through portion
- 140:: Extraction portion
- 160:: Needle part
- 200:: Accommodation unit
- 220:: First space
- 240:: Second space
- 242:: First partition portion
- 244:: Second partition portion
- 246:: Third partition portion
- 260:: Third space
- 280:: Opening/closing part
- 300:: Camera unit
- 320:: Image capturing part
- 340:: Adjustment part
- 342:: Rotary member
- 344:: Casing member
- 360:: Angle adjustment part
- 362:: Serrated member
- 364:: Distance member
- 400:: Power unit
- 420:: Battery part
- 440:: Terminal part

### [Mode for Invention]

Hereinafter, exemplary embodiments of the present invention for specifically accomplishing the objects of the present invention will be described with reference to the accompanying drawings.

In the description of the present embodiments, like terms and like reference numerals are used for like configurations, and additional descriptions for the like configurations will be omitted.

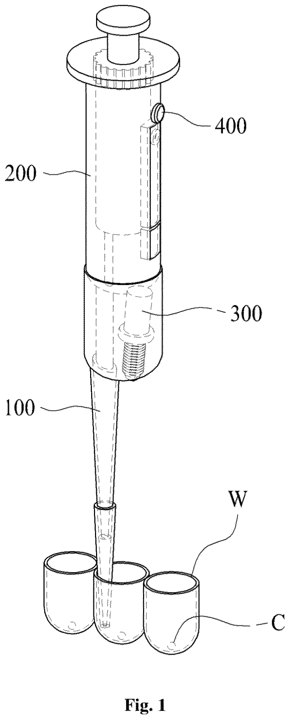

First, an endoscopic pipette according to an embodiment of the present invention will be described with reference to FIGS. 1 to 9.

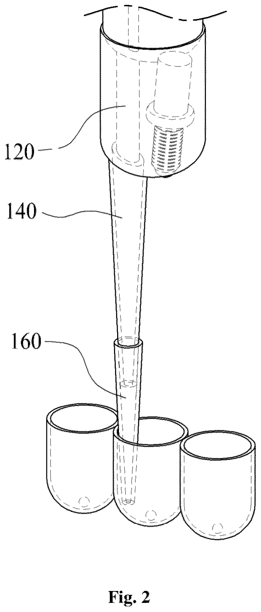

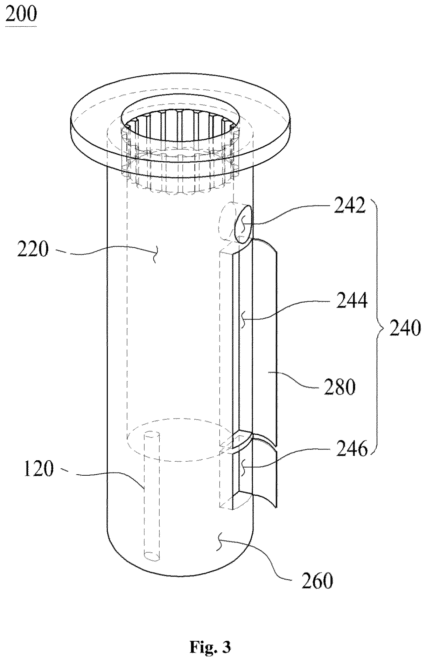

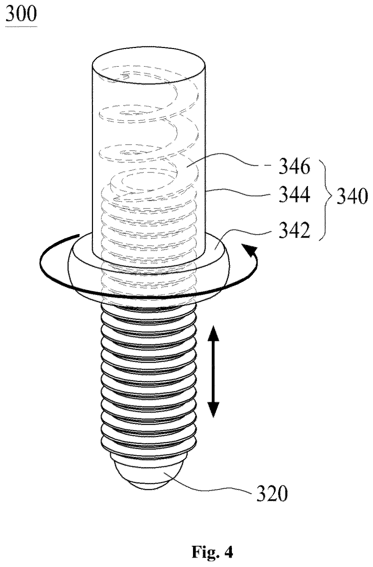

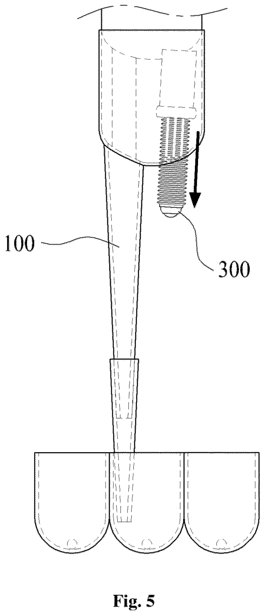

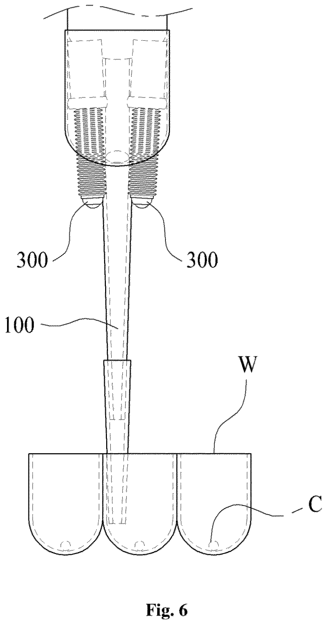

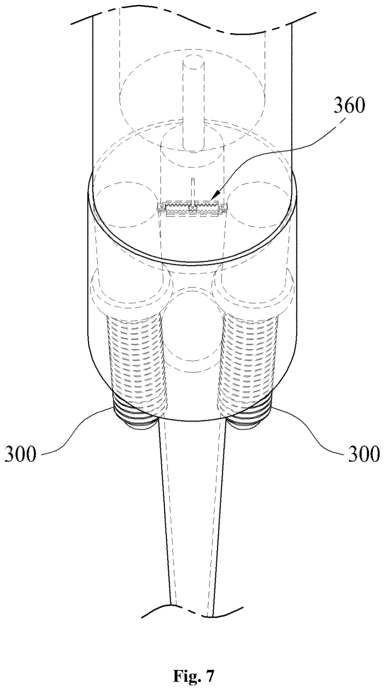

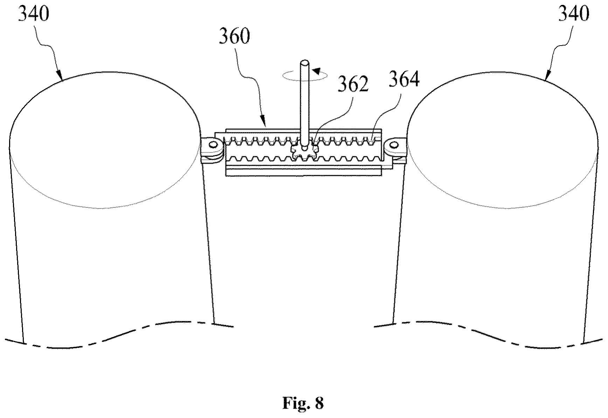

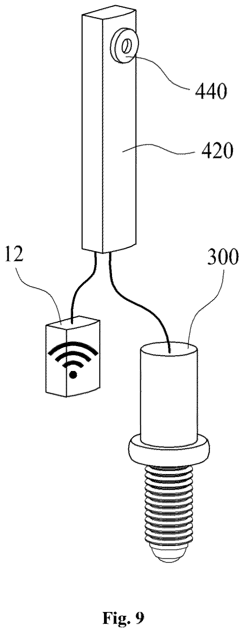

Specifically, FIG. 1 is a view for explaining an overall configuration of an endoscopic pipette and an observation device of an observation system according to an embodiment of the present invention, FIG. 2 is a view for explaining a tip unit of the endoscopic pipette and the observation system according to the embodiment of the present invention, FIG. 3 is a view for explaining an accommodation unit of the endoscopic pipette and the observation system according to the embodiment of the present invention, FIG. 4 is a view for explaining a camera unit of the endoscopic pipette and the observation system according to the embodiment of the present invention, FIG. 5 is a view for explaining an angle of a camera unit of the endoscopic pipette and the observation system according to the embodiment of the present invention, FIG. 6 is a view for explaining a pair of camera units of the endoscopic pipette and the observation system according to the embodiment of the present invention, FIG. 7 is a view for explaining an angle adjustment part of the endoscopic pipette and the observation system according to the embodiment of the present invention, FIG. 8 is a view for explaining a serrated member and a distance member of the endoscopic pipette and the observation system according to the embodiment of the present invention, and FIG. 9 is a view for explaining a power unit of the endoscopic pipette and the observation system according to the embodiment of the present invention.

First, as illustrated in FIG. 1, a pipette 10 may be used to observe spheroids C in a culture vessel W while removing a culture medium accommodated in the culture vessel W in which the spheroids C are cultured. The pipette 10 may include a tip unit 100 configured to be inserted into the culture vessel W to extract the culture medium, an accommodation unit 200 configured to accommodate the culture medium extracted by the tip unit 100, a camera unit 300 provided between the tip unit 100 and the accommodation unit 200, disposed adjacent to the tip unit 100, and configured to capture an image of an interior of the culture vessel W, and a power unit 400 provided in a partial space of the accommodation unit 200 and configured to provide power to the camera unit 300.

In this case, the tip unit 100 may be inserted into the culture vessel W to extract the culture medium in the culture vessel W. The tip unit 100 may be elongated from the accommodation unit 200 toward the culture vessel W.

In this case, as illustrated in FIG. 2, the tip unit 100 may include a through portion 120, an extraction portion 140, and a detachable portion 160.

In this case, the through portion 120 may be disposed to penetrate a third space 260 defined in the accommodation unit 200 and communicate with a first space 220 of the accommodation unit 200. This configuration will be described more specifically with respect to the drawings to be described below in detail.

Further, the extraction portion 140 may be elongated from the accommodation unit 200 toward the culture vessel W and extend so that a diameter thereof gradually decreases. This configuration is to adjust a flow velocity of the culture medium that moves to the accommodation unit 200. Because the spheroid C is too small to visibly identify, one end of the extraction portion 140 may be formed to be narrow to extract only the culture medium, except for the spheroids C.

Meanwhile, the spheroids C may be sucked and lost in case that a diameter of one end of the extraction portion 140 is relatively large even though the extraction portion 140 is inserted into the culture vessel W to extract the culture medium. In order to prevent this situation, the detachable portion 160 may be provided. The detachable portion 160 is formed to have a diameter corresponding to an outer diameter of the extraction portion 140 so that the extraction portion 140 is inserted into the detachable portion 160. The detachable portion 160 may be elongated toward the culture vessel W further than the extraction portion 140, such that one end of the detachable portion 160 has a smaller diameter than one end of the extraction portion 140.

This is to adjust a flow velocity and the amount of the culture medium sucked during the process of extracting the culture medium in the culture vessel W or to allow the detachable portion to have a smaller diameter than the extraction portion 140 to minimize a loss of the spheroids C.

That is, the detachable portion 160 may be formed to be detachable from one end of the extraction portion 140, and the extraction portion 140 and the detachable portion 160 may be formed to decrease in diameter toward one end. One end of the detachable portion 160 may be formed to have a relatively smaller diameter than one end of the extraction portion 140.

Meanwhile, as illustrated in FIG. 3, the accommodation unit 200 may be divided into the first space 220 configured to accommodate the culture medium extracted by the tip unit 100, a second space 240 in which the power unit 400 is positioned, and the third space 260 formed to surround the through portion 120.

In this case, the first space 220, the second space 240, and the third space 260 may be spaces, which are separated so as not to interfere with one another, i.e., independent spaces that do not interfere with one another.

In this case, because the first space 220 accommodates the culture medium extracted by the tip unit 100, the first space 220 may be formed to have a relatively larger volume than the second space 240 and the third space 260.

Meanwhile, the second space 240 may be disposed adjacent to the first space 220 and formed in a longitudinal direction of the accommodation unit 200.

Further, as illustrated in FIG. 3, the second space 240 may include a first partition portion 242, a second partition portion 244, and a third partition portion 246 that may respectively define spaces separated from one another.

In this case, a switch for operating a terminal part 440 of the power unit 400 or the camera unit 300 may be provided in the first partition portion 242.

In addition, a battery part 420, which provides electric power to operate the camera unit 300, may be disposed in the second partition portion 244. An opening/closing part 280 may be disposed in the second partition portion 244 and configured to selectively open or close the second partition portion 244 so that the battery part 420 may be replaced, as necessary.

Further, the opening/closing part 280 may also be disposed in the third partition portion 246. A transmission part 12 may be disposed in the third partition portion 246 and configured to transmit images, which are captured by the camera unit 300, to the outside. The camera unit 300, the power unit 400, the transmission part 12, and the like will be described more specifically with reference to the drawings.

Meanwhile, the third space 260 may provide a space in which the camera unit 300 is disposed. The third space 260 may be provided between the first space 220 and the second space 240 and between the second space 240 and the tip unit 100, and the through portion 120 may be disposed in the third space 260.

That is, the third space may be formed to surround a part of the tip unit 100 and define a space in which the camera unit 300 is disposed.

The above-mentioned configuration will be described simply. The accommodation unit 200 may be divided into two or more separate spaces and divided into the first space 220, the second space 240, and the third space 260 depending on the types of components accommodated therein. The first space 220 may accommodate the culture medium extracted by the tip unit 100, the second space 240 may accommodate the power unit 400, and the third space 260 may accommodate the camera unit 300 therein.

In addition, the second space 240 may be divided again into a plurality of separated spaces and divided into the first partition portion 242, the second partition portion 244, and the third partition portion 246 depending on the objects accommodated therein.

Meanwhile, the camera unit 300 may be provided in the third space 260 and disposed adjacent to the through portion 120. As illustrated in FIG. 4, the camera unit 300 may include an image capturing part 320 configured to capture images of the spheroids C in the culture vessel W, and an adjustment part 340 configured to adjust sizes of the spheroids C, which are to be observed, on a screen by adjusting a position of the image capturing part 320.

In this case, the image capturing part 320 may be connected to the transmission part 12 electrically or through near-field communication such as Bluetooth. The image capturing part 320 may use a visibly expandable lens such as an endoscope camera or a microscope in order to identify the spheroids C that are difficult to visibly identify.

Meanwhile, the adjustment part 340 may simply adjust the position of the image capturing part 320 to perform a zoom-in or zoom-out function. The adjustment part 340 may include a rotary member 342 configured to change a position of the image capturing part 320 while rotating, a casing member 344 having one end at which the rotary member 342 is positioned, the casing member 344 being configured such that a part of the image capturing part 320 is inserted into the casing member 344, and a spring member 346 having one end and the other end respectively coupled to the casing member 344 and the image capturing part 320, the spring member 346 being configured to transmit a rotational force of the rotary member 342 and move the image capturing part 320.

In this case, the rotary member 342 may be formed so that a part of the third space 260 is exposed to the outside. Therefore, a user may adjust a position of the image capturing part 320 by rotating the rotary member 342 exposed to the outside.

Further, a space may be defined in the casing member 344, and the image capturing part 320 may move in the casing member 344 and rotate in a rotation direction of the rotary member 342.

In addition, one end and the other end of the spring member 346 may be respectively connected to the image capturing part 320 and the casing member 344, and one end and the other end of the spring member 346 may move away from or toward each other in the rotation direction of the casing member 344 that rotates together with the rotary member 342 in the direction in which the rotary member 342 rotates, such that the image capturing part 320 may be moved, and the zoom-in or zoom-out function may be performed.

Further, as illustrated in FIG. 5, the image capturing part 320 may be provided in the third space 260 and inclined at a preset angle, and the preset angle may correspond to an inclination of an outer periphery of the tip unit 100.

This is to identify whether the spheroids C are sucked into the tip unit 100 in a direction in which one end of the tip unit 100 is directed.

That is, it is possible to identify a peripheral portion of one end of the tip unit 100.

Meanwhile, as illustrated in FIG. 6, in case that the camera unit 300 is provided as a pair of camera units 300 provided based on the through portion 120, the pair of camera units 300 may be provided to be inclined by the preset angle along the inclination of the tip unit 100 and capture images of one end of the tip unit 100.

This may be because it is generally more accurate to recognize an object with two visual fields than with a single visual field, and better able to can effectively determine distances and three-dimensions.

More specifically, in general, when perceiving objects with the single eye, there are problems such as unfamiliarity with the position and distance of the objects in comparison with the visual perception of the objects through the single eye, and a decrease in the sense of distance between the objects, as well as a decrease in the sense of three-dimensionality, which makes the size of the objects smaller than the tactile sense. In contrast, when utilizing binocular disparity, the stereoscopic and distance senses of the objects are higher than when using the single eye. Therefore, the pair of camera units 300 may be used to effectively observe and determine the interior of the culture vessel W in which the spheroids C are accommodated.

Meanwhile, because the observation accuracy is determined depending on an angle defined between the pair of camera units 300 in case that the pair of camera units 300 is used, the pipette may further include an angle adjustment part 360 configured to change an angle of the camera unit 300, as necessary.

In this case, as illustrated in FIG. 7, the angle adjustment part 360 may be disposed adjacent to the through portion 120 and provided between the pair of camera units 300.

Further, the angle adjustment part 360 may be coupled to ends of the pair of camera units 300, such that an angle at which the image capturing part 320 captures images may be adjusted by adjusting a distance between the ends of the pair of camera units 300.

More specifically, as illustrated in FIG. 8, the angle adjustment part 360 may be coupled to the casing member 340. The angle adjustment part 360 may include a serrated member 362 having one end protruding outward, like the rotary member 342, and the other end having a plurality of protrusions along a periphery thereof, and a pair of distance members 364 provided with the serrated member 362 interposed therebetween, the pair of distance members 364 having protrusions, which have shapes corresponding to the periphery of the serrated member 362, and configured to be moved by a rotation of the serrated member 362.

When one end of the serrated member 362 is rotated, the pair of distance members 364 may be moved in opposite directions by means of the protrusions that engage with one another in response to the rotation of the serrated member 362, such that the distance between the ends of the camera units 300 may be adjusted.

As described above, the binocular disparity may be more easily used by adjusting angles at which the camera units 300 capture images by adjusting the distance between the pair of camera units 300. Therefore, it is possible to more assuredly identify whether the spheroids C are lost during the process of extracting the culture medium by means of the tip unit 100.

Meanwhile, as illustrated in FIG. 9, the power unit 400 may include the battery part 420 positioned in the second space 240, elongated in one direction, and configured to provide electric power to the camera unit 300, the terminal part 440 connected to an external terminal to supply electric power to the battery part 420, and the transmission part 12 configured to transmit images, which are captured by the camera units 300, to the outside.

In this case, as described above, the battery part 420 may be disposed to be replaceable and replaced by the opening/closing part 280 configured to selectively open the second partition portion 244. The battery part 420 may be electrically connected to the camera unit 300 and the transmission part 12.

Further, a switch part may be provided to selectively operate the battery part 420. As described above, the switch part may be provided in the first partition portion 242 and electrically connected to the battery part 420.

Meanwhile, the terminal part 440 may mean a position into which a terminal is inserted to charge the battery part 420, which has consumed electric power, with electric power in a state in which the battery part 420 is replaced. The terminal part 440 may be provided at a part of a periphery of the battery part 420.

Meanwhile, the transmission part 12 may be electrically connected to the image capturing part 320. However, as described above, the transmission part 12 may be connected to the image capturing part 320 through near-field communication such as Bluetooth and transmit images, which are captured by the image capturing part 320, to the outside.

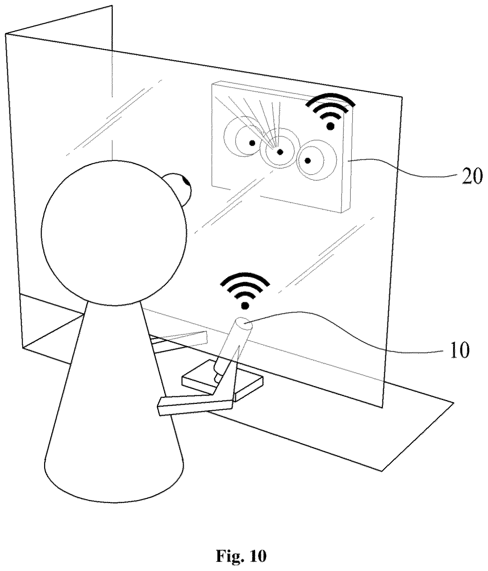

In order to more specifically describe the above-mentioned configuration, an observation system according to the embodiment of the present invention may be described with reference to FIG. 10.

Specifically, FIG. 10 is a view for explaining the endoscopic pipette and the observation system according to the embodiment of the present invention.

In this case, as illustrated in FIG. 10, the user may extract the culture medium or observe the spheroids C by using the pipette 10 including the tip unit 100, the accommodation unit 200, the camera unit 300, and the power unit 400. When the images captured by the image capturing part 320, which captures images of the interior of the culture vessel W, are transmitted to the transmission part 12 through wireless communication, the transmission part 12 may transmit the images, which are captured by the image capturing part 320, to the outside.

In this case, the observation system of the present invention may include the pipette 10 and a display 20 configured to display the images transmitted from the transmission part 12, and the images transmitted from the transmission part 12 are enlarged and displayed on the display 20, such that the user may more effectively identify the images visibly.

Further, like the transmission part 12 connected to the camera unit 300 electrically or through wireless communication, the transmission part 12 and the display 20 may be connected to each other electrically or through near-field communication. For example, the transmission part 12 and the display 20 may be connected to each other through Bluetooth.

However, the electrical connection may affect a movement range of the pipette 10 and cause an unexpected accident in which the culture vessel W is touched by an electric wire or the like used to electrically connect the pipette 10 and the display 20. The connection through wireless communication may be preferable in comparison with the electrical connection.

Meanwhile, as described above, in case that the pair of camera units 300 is provided, the display 20 may be equipped with a correction unit for correcting the images transmitted from the transmission part 12. The correction unit may three-dimensionally correct the images captured by the different camera units 300 into a single image and display the single image on the display 20.

That is, the correction unit may edit and correct the plurality of images transmitted from the transmission part 12 and exhibit an effect equivalent to viewing the culture vessel W with a single camera. Therefore, the user may visibly identify the spheroids C, which is difficult to visibly identify, and identify the motions of the spheroids C during the process of extracting the culture medium, which may effectively prevent a loss of the spheroids C.

While the exemplary embodiments according to the present invention have been described above, it is obvious to those skilled in the art that the present invention may be specified in other particular forms in addition to the aforementioned embodiments without departing from the spirit or the scope of the present invention.

Accordingly, it should be understood that the aforementioned embodiments are not restrictive but illustrative, and thus the present invention is not limited to the aforementioned description, and may be modified within the scope of the appended claims and the equivalent range thereto.

## Claims

1. An endoscopic pipette, which is configured to remove a culture medium accommodated in a culture vessel, in which spheroids are cultured, and observe the spheroids in the culture vessel, the endoscopic pipette comprising:
a tip unit configured to be inserted into the culture vessel to extract the culture medium;
an accommodation unit configured to accommodate the culture medium extracted by the tip unit;
a camera unit provided between the tip unit and the accommodation unit, disposed adjacent to the tip unit, and configured to capture an image of an interior of the culture vessel; and
a power unit provided in a partial space of the accommodation unit and configured to provide power to the camera unit.

2. The endoscopic pipette of claim 1, wherein the accommodation unit is divided into a first space in which the culture medium introduced through the tip unit is accommodated, a second space in which the power unit is positioned, and a third space in which the camera unit is positioned.

3. The endoscopic pipette of claim 2, wherein the second space is disposed adjacent to the first space in which the culture medium is accommodated, and the second space is elongated in a longitudinal direction of the accommodation unit.

4. The endoscopic pipette of claim 3, wherein the power unit comprises:
a battery part configured to store electric power and provide the electric power to the camera unit;
a switch part configured to operate the battery part; and
a transmission part provided in the second space, disposed adjacent to the camera unit, and configured to transmit the image captured by the camera unit to the outside.

5. The endoscopic pipette of claim 4, wherein the second space is divided into three or more spaces so that the battery part, the switch part, and the transmission part are positioned in the separated spaces.

6. The endoscopic pipette of claim 5, wherein the defined space of the second space, in which the battery part is positioned, is formed to be openable and closable so that the battery part is replaced depending on the amount of electric power remaining in the battery part.

7. The endoscopic pipette of claim 2, wherein the third space is disposed below the first space, and the tip unit is coupled to penetrate the third space toward the first space.

8. The endoscopic pipette of claim 7, wherein the tip unit comprises:
a through portion configured to penetrate the third space and having a constant diameter; and
an extraction portion extending to the outside of the third space so that a diameter thereof decreases from the through portion toward the culture vessel, the extraction portion being inserted into the culture vessel to extract the culture medium.

9. The endoscopic pipette of claim 2, wherein the camera unit comprises:
an image capturing part configured to capture an image of the interior of the culture vessel; and
an adjustment part in which the image capturing part is positioned, the adjustment part being configured to move the image capturing part in a rotation direction.

10. The endoscopic pipette of claim 9, wherein the adjustment part comprises:
a rotary member formed to surround a periphery of the image capturing part and partially exposed to the outside of the third space, the rotary member configured to move the image capturing part while rotating;
a casing member having one end at which the rotary member is positioned, the casing member being configured to rotate in a rotation direction of the rotary member and configured such that a part of the image capturing part is inserted into the casing member; and
a spring member having one end and the other end respectively coupled to the image capturing part and the casing member, the spring member being configured to move the image capturing part toward the culture vessel in accordance with the rotation of the rotary member.

11. The endoscopic pipette of claim 10, wherein the camera unit is provided as a pair of camera units positioned with the tip unit interposed therebetween.

12. The endoscopic pipette of claim 11, wherein the camera unit comprises an angle adjustment part positioned between the pair of adjustment parts and configured to change an angle of the image capturing part while changing a distance between the other ends of the adjustment parts.

13. The endoscopic pipette of claim 12, wherein the angle adjustment part comprises:
a serrated member having one end positioned outside the third space, and the other end positioned in the third space, the serrated member having a plurality of protrusions formed along a periphery of the other end; and
a pair of distance members provided with the serrated member interposed therebetween, the pair of distance members having grooves corresponding to the protrusions of the serrated member and configured to be moved by a rotation of the serrated member.

14. An observation system, which is configured to observe spheroids accommodated in a culture vessel, the observation system comprising:
the endoscopic pipette of any one of claims 1 to 13; and
a display configured to receive an image captured by the endoscopic pipette and display the image.

15. The observation system of claim 14, wherein the endoscopic pipette transmits the image, which is captured by the image capturing part, to the display through the transmission part.

16. The observation system of claim 15, wherein the transmission part is connected to the display through Bluetooth.

17. The observation system of claim 15, comprising:
a correction unit configured to correct a pair of images captured by a pair of image capturing parts so that the pair of images is visibly observed through binocular disparity.
